(19) Europäisches Patentamt
European Patent Office
Office européen des brevets

(11) EP 1 317 264 B1

(12) **EUROPÄISCHE PATENTSCHRIFT**

(45) Veröffentlichungstag und Bekanntmachung des Hinweises auf die Patenterteilung:
**28.07.2004 Patentblatt 2004/31**

(21) Anmeldenummer: **01963002.9**

(22) Anmeldetag: **11.09.2001**

(51) Int Cl.[7]: **A61K 31/385**, A23L 1/30, A61P 3/10 // (A61K31/385, 31:20)

(86) Internationale Anmeldenummer:
**PCT/EP2001/010501**

(87) Internationale Veröffentlichungsnummer:
**WO 2002/022111 (21.03.2002 Gazette 2002/12)**

(54) **KOMBINATION VON LIPONSÄURE UND KONJUENSÄUREN ZUR BEHANDLUNG DIABETISCHER STÖRUNGEN**

COMBINATION OF LIPOIC ACIDS AND CONJUGATED ACIDS FOR TREATING DIABETIC DISORDERS

COMBINAISON D'ACIDES LIPOIQUES ET D'ACIDES GRAS A DOUBLES LIAISONS POUR TRAITER LE DIABETE

(84) Benannte Vertragsstaaten:
**AT BE CH CY DE DK ES FI FR GB GR IE IT LI LU MC NL PT SE TR**
Benannte Erstreckungsstaaten:
**RO SI**

(30) Priorität: **12.09.2000 DE 10045059**

(43) Veröffentlichungstag der Anmeldung:
**11.06.2003 Patentblatt 2003/24**

(73) Patentinhaber: **BASF AKTIENGESELLSCHAFT**
**67056 Ludwigshafen (DE)**

(72) Erfinder:
- **HASSELWANDER, Oliver**
  **76829 Landau (DE)**
- **KRÄMER, Klaus**
  **76829 Landau (DE)**
- **BALDENIUS, Kai-Uwe**
  **67063 Ludwigshafen (DE)**
- **KLATT, Martin, Jochen**
  **67098 Bad Dürkheim (DE)**
- **SCHWEIKERT, Loni**
  **67122 Altrip (DE)**

(74) Vertreter: **Wolter, Thomas, Dr. et al**
**Reitstötter, Kinzebach & Partner (GbR)**
**Patentanwälte**
**Postfach 86 06 49**
**81633 München (DE)**

(56) Entgegenhaltungen:
**WO-A-99/29317** **US-A- 5 948 810**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 317 264 B1

## Beschreibung

Die vorliegende Erfindung betrifft die Verwendung von Liponsäure und Konjuensäuren zur Nahrungsergänzung, in funktionalen Lebensmitteln (functional foods) und zur Herstellung eines Mittels zu therapeutischen Zwecken bei der Behandlung diabetischer Störungen, Mittel mit einer entsprechenden Wirkstoffkombination sowie Mittel in Form von Handelspackungen mit entsprechenden Kombinationspräparaten oder Monopräparaten zur kombinierten Anwendung.

Neuere Schätzungen gehen weltweit von etwa 135 Mio. an Diabetes mellitus erkrankten Patienten aus. Bis zum Jahr 2025 wird mit einem Anstieg auf etwa 300 Mio. gerechnet. Allein in den USA leiden etwa 15,7 Mio. Menschen, das sind 5,9 % der Bevölkerung an Diabetes mellitus. Darunter befinden sich etwa 5,4 Mio., deren Erkrankung noch nicht diagnostiziert wurde.

Diabetes mellitus, üblicherweise auch als Zuckerkrankheit bezeichnet, ist eine Störung des Kohlenhydratstoffwechsels. Im Wesentlichen begegnet man zwei Arten dieser Erkrankung: Diabetes Typ I infolge Insulinsmangels und daher auch als Insulin-abhängiger Diabetes mellitus (IDDM) bezeichnet, und Diabetes Typ II infolge verminderter Insulinwirkung, daher auch als nicht-Insulinabhängiger Diabetes mellitus (NIDDM) bezeichnet. Typ I manifestiert sich in der Regel bei Kindern und Jugendlichen und bedarf zur Behandlung einer lebenslangen Insulinzufuhr. Mehr als 90 % aller Fälle betreffen allerdings den Typ II, der sich in der Regel im Erwachsenenalter manifestiert. Die Behandlung dieses im Wesentlichen auf eine Insulinresistenz zurückzuführenden Typs erfolgt gewöhnlicherweise mit einer speziellen Diabeteskost und Antidiabetika bei regelmäßiger Kontrolle des Blutzuckerspiegels. Nur in seltenen Fällen ist eine Behandlung des Typs II mit Insulin angezeigt.

Die wichtigsten zur symptomatischen Hyperglykämie-Behandlung eingesetzten Wirkstoffe gliedern sich in hypoglykämische Wirkstoffe, zu denen vor allem die Sulfonylharnstoffe gehören, und antihyperglykämische Wirkstoffe, zu denen die Biguanidine, die $\alpha$-Glucosidase-Inhibitoren und die Insulinsensibilisatoren, insbesondere die Thiazolidindione, gehören.

Sulfonylharnstoffe, wie Tolbutamid, Chlorpropamid, Glyburid und ähnliche Sulfonylharnstoffe der ersten und zweiten Generation, senken den Plasmaglucosespiegel in erster Linie dadurch, dass sie die Insulinsekretion stimulieren. Problematisch ist das damit verbundene hohe Risiko, eine Sulfonylharnstoff-induzierte Hypoglykämie zu entwikkeln, weshalb viele, vor allem ältere Patienten hospitalisiert werden müssen.

Biguanide, wie Metformin senken die hepatische Glucoseproduktion. Bei Patienten mit Nieren- oder Leberstörungen und in Fällen von Alkoholismus sind diese Wirkstoffe allerdings kontraindiziert. Im übrigen werden häufig gastrointestinale Nebenwirkungen beobachtet.

$\alpha$-Glucosidase-Inhibitoren, wie Acarbose, hemmen kompetitiv die Hydrolyse von Sacchariden. Auch diese Wirkstoffe verursachen häufig unerwünschte Nebenwirkungen.

Thiazolidindione, wie Troglitazon verbessern die Insulinempfindlichkeit des Skelettmuskels und vermindern den hepatischen Glucoseausstoß. Problematisch ist allerdings die mögliche idiosynkratische Lebertoxizität dieser Wirkstoffe. So besteht trotz verschiedenster zur Verfügung stehender Antidiabetika ein Bedarf für weitere Behandlungsmöglichkeiten von Diabetes mellitus.

Als Coenzym bei der oxidativen Decarboxylierung von $\alpha$-Ketosäuren findet man Liponsäure in nahezu jeder Zelle eines Organismus. Antiphlogistische, analgetische und cytoprotektive Eigenschaften wie auch ihre antioxidative Wirkung machen die Liponsäure zu einem interessanten Wirkstoff für Pharmazie, Kosmetik, Ernährungswissenschaft und angrenzende Gebiete (Biothiols in Health and Disease, Herausgeber Packer L. und Cadenas E., Marcel Dekker Inc., New York, Basel, Hongkong). So wird über verschiedene Studien an diabetischen Patienten berichtet, bei denen die Verabreichung von Liponsäure Wirkung zeigte. Beispielsweise beschreiben Jacob et al., Arzneim.-Forsch./Drug Res. 45 (II) Nr. 8 (1995) 872-874 eine deutliche Verbesserung der Glucose-Verwertung von Patienten mit Diabetes Typ II nach einmaliger parenteraler Gabe von 1000 mg Liponsäure. Ähnliche Ergebnisse wurden für eine chronische parenterale Gabe berichtet (Jacob et al., Exp. Clin. Endocrinol. Diabetes 104 (1996) 284-288). In einer Studie zur Behandlung diabetischer Neuropathie mit Liponsäure (ALADIN) nahmen symptomatische Beschwerden bei 3-wöchiger intravenöser Verabreichung von täglich 600 mg Liponsäure ab (Ziegler et al. Diabetologia (1995) 38: 1425-1433). Kürzlich konnte in einer Fortsetzung dieser Studie zur symptomatischen Behandlung diabetischer Polyneuropathie mit Liponsäure (ALADIN III) allerdings keine von Placebo unterscheidbare Wirkung auf neuropathische Symptome festgestellt werden, wenngleich die 3-wöchige intravenöse und anschließende 6-monatige orale Behandlung mit Liponsäure neuropathische Defizite in günstiger Weise zu beeinflussen schien (Ziegler et al., Diabetes Care 22: 1296-1301, 1999). In der DEKAN-Studie konnte keine signifikante Veränderung cardiovaskulärer autonomer Symptome an NIDDM-Patienten beobachtet werden, die 4 Monate täglich 800 mg Liponsäure bekamen (Ziegler et al., Diabetes Care 20 (1997) 369-373). In einer neueren Multicenter-Studie an Patienten mit Diabetes Typ II wurde festgestellt, dass eine 1- bis 3-malige tägliche orale Verabreichung von 600 mg Liponsäure die Insulinempfindlichkeit beeinflussen konnte (Jacob et al., Free Radical Biology & Medicine, Vol. 27, Nos. 3/4, 309-314, 1999 und BioFactors 10 (1999) 169-174).

In Deutschland sind Liponsäure-haltige Präparate derzeit zur Behandlung von Mißempfindungen bei diabetischer Polyneurapathie gelistet. Formulierungen fester Salze von Liponsäure werden in der US-A-5,990,152 vorgeschlagen. Die US-A-5,994,393 betrifft eine weitere Modifikation von Liponsäure. Brauchbare Liponsäure-Analoga werden in der WO 99/45922 vorgeschlagen. Kombinationen von Liponsäure und Vitaminen zur Herstellung von Arzneimitteln werden in der EP 0 572 922 A1 beschrieben. Eine Kombination von Liponsäure als biokompatibles Disulfid mit einer essentiellen Fettsäure, insbesondere gamma-Linolensäure, Arachidonsäure, Stearidonsäure, Eicosapentaensäure und Docosahexaensäure, und gegebenenfalls weiteren essentiellen Nährstoffen, u.a. zur Behandlung von Diabetes und diabetischen Komplikationen, wird in der WO 99/04782 angesprochen. Speziell diabetische Neuropathien und andere Langzeit-Komplikationen diabetogener Genese sollen gemäß EP 0 218 460 mit gamma-Linolensäure bzw. deren Metaboliten behandelbar sein.

Neben essentiellen Fettsäuren können allerdings auch weitere, an sich nicht essentielle Fettsäuren für den menschlichen oder tierischen Organismus von Bedeutung sein. Beispielsweise wurde den bereits Ende der 80'iger Jahre entdeckten konjugierten Linolsäuren antikarzinogene, antiarteriosklerotische und immunstimulierende Wirksamkeit zugeschrieben. Es handelt sich hierbei um eine Gruppe von Linolsäureisomeren, z.B. mit zwei an Position 9 und 11 oder 10 und 12 sitzenden konjugierten Doppelbindungen. Kürzlich wurde in der WO 99/29317 die Verwendung konjugierter Linolsäuren zur Behandlung von Diabetes vorgeschlagen.

Es wurde nun gefunden, daß bestimmte kombinierte Anwendungen von Liponsäuren mit Konjuensäuren eine effektive Behandlungsmöglichkeit diabetischer Störungen eröffnen und damit eine ideale Nahrungsergänzung darstellen, Lebensmitteln einen positiven Einfluss auf die Gesundheit verleihen und einen hohen therapeutischen Wert besitzen.

Gegenstand der vorliegenden Erfindung ist daher die Verwendung wenigstens einer Liponsäure, eines physiologisch akzeptablen Derivates oder Salzes davon und wenigstens einer Verbindung der Formel I

$$H_3C\text{-}(CH_2)_{n1}\text{-}(CH{=}CH)_{n3}\text{-}(CH_2)_{n2}\text{-}COOH \qquad \text{I}$$

worin n1, n2 unabhängig voneinander den Wert einer ganzen Zahl von 3 bis 9 haben, n3 den Wert einer ganzen Zahl von 2 bis 6, vorzugsweise 2 oder 3, hat und die Gesamtanzahl der Kohlenstoffatome 18, 20 oder 22, vorzugsweise 18, beträgt eines physiologisch akzeptablen Derivates oder Salzes davon, zur Herstellung eines Mittels zur Behandlung diabetischer Störungen.

Die erfindungsgemäße Verwendung stellt eine Kombinationstherapie dar, d.h. die Verwendung von Liponsäuren, physiologisch akzeptabler Derivate oder Salze davon - im Folgenden zwecks Vereinfachung auch als "Liponsäurekomponente" bezeichnet - und die Verwendung von Verbindungen der Formel I, physiologisch akzeptabler Derivate oder Salze davon - im folgenden zwecks Vereinfachung auch als CA-Komponente (für Conjugated Acid-Komponente) bezeichnet - erfolgt in einem insbesondere therapeutisch angemessenem Zusammenhang, vor allem mit Blick auf optimale Wirksamkeit. So können die Liponsäure-Komponente und die CA-Komponente prinzipiell gemeinsam in einer Formulierung oder getrennt in wenigstens zwei verschiedenen Formulierungen verabreicht werden. Die letztere Möglichkeit beinhaltet sowohl die gleichzeitige, d.h. zu im Wesentlichen gleichen Zeitpunkten erfolgende oder unmittelbar aufeinanderfolgende, Verabreichung, als auch die zeitlich beabstandete, d.h. zu unterschiedlichen Zeitpunkten erfolgende, Verabreichung. Eine besondere Ausführungsform der zeitlich beabstandeten Verabreichung wird durch die abwechselnde Verabreichung beider Komponenten, beispielsweise mit einem Früh/Spät-Tagesrhythmus, realisiert.

Somit betrifft die vorliegende Erfindung sowohl die Verwendung wenigstens einer Liponsäure, eines physiologisch akzeptablen Derivates oder Salzes davon und die Verwendung wenigstens einer Verbindung der Formel I, eines physiologisch akzeptablen Derivates oder Salzes davon, zur Herstellung eines Mittels zur CA-unterstützten Behandlung bzw. zur Liponsäure-unterstützten Behandlung diabetischer Störungen. In diesem Sinne sind Gegenstand der Erfindung Mittel zur Behandlung diabetischer Störungen, die auf einer Kombination aus wenigstens einer Liponsäure, eines physiologisch akzeptablen Derivates oder Salzes davon und wenigstens einer Verbindung der Formel I, eines physiologisch akzeptablen Derivates oder Salzes davon, sowie gegebenenfalls weiteren Wirkstoffen basieren, wobei die Wirkstoffkomponenten, insbesondere die Liponsäure- und CA-Komponente, gemeinsam oder getrennt formuliert sein können.

Der Begriff "Liponsäure" bezeichnet erfindungsgemäß 5-(1,2-Dithiolan-3-yl)valeriansäure, auch Thioctsäure, Thioctansäure oder Thioctinsäure genannt, der Formel II

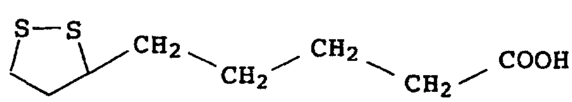

II

die unter diese Formel fallenden optischen Isomere sowohl als Gemische, z.B. Racemate, als auch in Reinform, z.B. R- oder S-Enantiomere, eingeschlossen. Das bevorzugte Isomer ist die (R)-5-(1,2-Dithiolan-3-yl)valeriansäure der Formel III

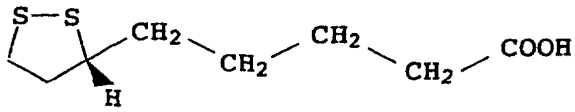

III

Bevorzugt sind Liponsäure-Gemische mit einem (R)-Enantiomeren-Überschuß (ee) von mindestens 40%. Vorzugsweise liegt der (R)-Enantiomeren-Überschuß bei mindestens 80%, insbesondere mindestens bei 98%.

Zu Liponsäurederivaten gehören insbesondere Synthesevorstufen und Metabolite der Liponsäure, also vor allem Dihydroliponsäure. Als weitere Metabolite sind Liponamid, Lipoyllysin, Di-6,8-bis-norliponsäure und Tetranorliponsäure zu nennen. Weitere geeignete Liponsäurederivate sind beispielsweise die in der WO 99/45922 als Liponsäure-Analoga der Formel (I) beschriebenen Ester, Thioester und Amide von Liponsäure mit Aminoalkoholen, Aminothiolen bzw. Diaminen, welche durch Bezugnahme Teil der vorliegenden Anmeldung sind. Den Ausführungen zur Liponsäure entsprechend gehören auch die jeweiligen optischen Isomere der Derivate dazu.

Die physiologisch akzeptablen Salze von Liponsäure bzw. Liponsäurederivaten sind im vorliegenden Fall bevorzugt Basenadditionssalze.

Zu den Basenadditionssalzen zählen Salze mit anorganischen Basen, beispielsweise Metallhydroxiden bzw. -carbonaten von Alkali-, Erdalkali- oder Übergangsmetallen, oder mit organischen Basen, beispielsweise Ammoniak oder basischen Aminosäuren, wie Arginin und Lysin, Aminen, z.B. Methylamin, Dimethylamin, Trimethylamin, Triethylamin, Ethylamin, Diethylamin, Ethylendiamin, Ethanolamin, Diethanolamin, 1-Amino-2-propanol, 3-Amino-l-propanol oder Hexamethylentetramin, gesättigten cyclischen Aminen mit 4 bis 6 Ringkohlenstoffatomen, wie Piperidin, Piperazin, Pyrrolidin und Morpholin, sowie weiteren organischen Basen, beispielsweise N-Methylglucamin, Kreatin und Tromethamin, sowie quaternären Ammoniumverbindungen, wie Tetramethylammonium und dergleichen.

Bevorzugt werden Salze mit anorganischen Basen, z.B. Na-, K-, Mg-, Ca-, Zn-, Cr- und Fe-Salze.

Die Verbindungen der Formel I gehören zu den mehrfach ungesättigten Fettsäuren mit konjugierten Doppelbindungen (Konjuensäuren). Insbesondere zählen hierzu Verbindungen der Formel IV

$$H_3C\text{-}(CH_2)_{n1}\text{-}CH{=}CH\text{-}CH{=}CH\text{-}(CH_2)_{n2}\text{-}COOH \qquad IV$$

worin n1, n2 unabhängig voneinander den Wert einer ganzen Zahl von 3 bis 9 haben und die Summe aus (n1+n2)=12 beträgt. Diese weisen wie Linolsäure 18 Kohlenstoffatome und 2 Doppelbindungen auf ($C_{18}$:2). Im Unterschied zur Linolsäure sind die Doppelbindungen konjugiert und können neben der in Linolsäure vorliegenden cis-Konfiguration auch trans-Konfiguration aufweisen. Diese als konjugierte Linolsäuren (CLA) zu bezeichenenden Verbindungen der Formel IV stellen daher Isomere der Linolsäure dar, die sich durch Position und/oder cis,trans-Konfiguration von dieser essentiellen Fettsäure unterscheiden. Demnach handelt es sich um Positionsisomere und/oder geometrische Isomere. Zu weiteren brauchbaren Konjuensäuren gehört insbesondere Calendulasäure (8t,10t,12c-Octadecatriensäure).

Zu Verbindungen der Formel IV gehören insbesondere Octadecadi-8,10-ensäuren (n1,n2 = 6), Octadecadi-9,11-ensäuren (n1 = 5; n2 = 7), Octadecadi-10,12-ensäuren (n1 = 4; n2 = 8) und Octadecadi-11,13-ensäuren (n1 = 3; n2 = 9). Von diesen Isomeren kommen die 9,11- und 10,12-Isomere bevorzugt zum Einsatz.

Was die geometrische Konfiguration angeht, so werden cis, trans- und trans,cis-Isomere gegenüber cis,cis- und trans,trans-Isomeren bevorzugt, beispielsweise 9c,11t- und 9t, 11c-Isomere gegenüber 9c,11c- und 9t,11t-Isomeren beziehungsweise in entsprechnder Anwendung die cis,trans- und trans,cis-Isamere der 8,10-, 10,12- und 11,13-Diensäuren.

Gemäß einer besonderen Ausführungsform enthält die CA-Komponente wenigstens eine Verbindung der Formel IV, die ausgewählt ist unter den 9c,11t- und 10t,12c-Isomeren der Octadecadiensäure.

Die Verwendung eines Isomers als CA-Komponente kann zweckmäßig sein. Andererseits kann es aus praktischen Gründen vorteilhaft sein, zwei oder mehrere der zuvor genannten Isomere zu verwenden. Dabei können sich die Isomere in Position und/oder geometrischer Konfiguration ihrer Doppelbindungen voneinander unterscheiden. Der Anteil an 9,11- und/oder 10,12-Isomeren am Gesamtgewicht der CA-Komponente beträgt vorteilhafterweise wenigstens 50 Gew.-%, vorzugsweise wenigstens 70 Gew.-% und insbesondere wenigstens 90 Gew.-%. Einem anderen vorteilhaften Aspekt zufolge beträgt der Anteil an anderen Verbindungen der Formel IV als den 9,11- und/oder 10,12-Isomeren jeweils weniger als 5 Gew.-%, vorzugsweise weniger als 2 Gew.-% und insbesondere weniger als 1,5

Gew.-%. Gemäß einer Ausführungsform der vorliegenden Erfindung werden 9,11- und 10,12-Isomere in Kombination verwendet. Es können dabei auch Anteile an 8,10- und/oder 11,13-Isomeren mitverwendet werden. In diesem Fall ist es bevorzugt, verhältnismäßig größere Mengen an 9,11- und 10,12-Isomeren als 8,10- und/oder 11,13-Isomeren zu verwenden. Vorteilhafterweise verhält sich die Gesamtmenge aus 9,11- und 10,12-Isomeren zur Gesamtmenge aus 8,10- und 11,13-Isomeren wenigstens wie 3:2, vorzugsweise wenigstens wie 10:1 und insbesondere wenigstens 50: 1. Besonders bevorzugt beträgt der Anteil an 8,10- und/oder 11,13-Isomeren jeweils weniger als 1 Gew.-%, bezogen auf das Gesamtgewicht der CA-Komponente.

Gemäß einer besonderen Ausführungsform werden CA-Kombinationen verwendet, die im Wesentlichen aus 9,11- und 10,12-Isomeren gebildet werden. Dazu kann man das Verhältnis von 9,11- und 10,12-Isomeren zu einem Restanteil an 8,10- und/oder 11,13-Isomeren größer 9:1 vorzugsweise größer 9,5:0,5 und insbesondere größer 9,9: 0,1 wählen.

Im Rahmen der vorstehend beschriebenen Ausführungsform kann jedes der Positionsisomere eines oder mehrere der möglichen geometrischen Isomere beinhalten. So können tatsächlich alle 16 Isomere, insbesondere im Rahmen der vorstehend beschriebenen bevorzugten und vorteilhaften Verteilungen einzelner Positionsisomere, verwendet werden. Hierbei überwiegt vorteilhafterweise der jeweilige Anteil an cis,trans- und trans,cis-Isomeren im Verhältnis zu cis,cis- und/oder trans,trans-Isomeren. Bevorzugt werden Verhältnisse von cis,trans- und trans,cis-Isomeren zu cis,cis- und/oder trans,trans-Isomeren von wenigstens 5:1, vorzugsweise von wenigstens 10:1 und insbesondere von wenigstens 25:1.

Die kombinierte Verwendung mehrerer Isomere erfolgt in der Regel als Gemisch.

Zu Derivaten von Verbindungen der Formel I gehören insbesondere Ester, die unter physiologischen Bedingungen hydrolysierbar sind, sowie Metabolite dieser Verbindungen, insbesondere 3-fach, 4-fach, 5-fach oder 6-fach ungesättigte und gegebenenfalls um 2 oder 4 Kohlenstoffatome verlängerte Derivate von Verbindungen der Formel IV. Derartige Derivate könnnen beispielsweise unter Einwirkung von Desaturasen und/oder Elongasen erhalten werden. Auch hydroxylierte Formen von Verbindungen der Formel I gehören zu den Derivaten. Bevorzugte Ester basieren auf Verbindungen der Formel I mit Alkoholen, die auch in natürlich vorkommenden Fettsäureestern zu finden sind, oder mit $C_1$-$C_8$-, vorzugsweise $C_2$-$C_4$-Alkanolen. So sind von den Esterderivaten der Verbindungen der Formel I insbesondere Glyceride und Phospholipide zu nennen, wobei in den Tri- und Diglyceriden sowie in Phospholipiden ein oder zwei nicht unter die Formel I fallende Säurereste gebunden sein können. Von den Alkylestern sind insbesondere die Ethylester zu nennnen. Im übrigen gelten für die Derivate obige Ausführungen zu den Isomeren entsprechend.

Die physiologisch akzeptablen Salze von Verbindungen der Formel I bzw. von Derivaten davon sind im vorliegenden Fall bevorzugt Basenadditionssalze, insbesondere die in Zusammenhang mit Liponsäure angegebenen Salze.

Verbindungen der Formel I können in an sich bekannter Weise hergestellt werden. Einige von ihnen sind auch käuflich erhältlich. Vielfach handelt es sich um Gemische isomerer Verbindungen der Formel I, deren Zusammensetzung je nach Art der Herstellung und des Ausgangsproduktes sowie gegebenenfalls vorgenommener Maßnahmen zur Anreicherung und/oder Abtrennung eines oder mehrerer Isomere unterschiedlich sein können. Ferner können derartige Gemische auch weitere Bestandteile, insbesondere weitere Fettsäuren, vor allem Linolsäure, Ölsäure, Palmitinsäure, Stearinsäure und weitere Lipide oder Lipoide umfassen.

Beispielsweise können Verbindungen der Formel I und insbesondere Verbindungen der Formel IV aus den Nicht-Konjuensäuren, insbesondere Linolsäure oder linolsäurehaltigen Gemischen, hergestellt werden. Es sind chemische und auch enzymatische Verfahren bekannt. Beispielhaft seien Isomerisierungsreaktionen von Nicht-Konjuensäuren, insbesondere Linolsäure, unter alkalischen Bedingungen oder mittels enzymatischer Katalyse durch Transisomerasen, beispielsweise der Transisomerase aus Butyrivibrio fibrisolvens, genannt. Auch fermentative Prozesse, z.B. eine Fermentation von Lactobacillus, Clostridium und Bifidobakterien, sind geeignet.

Linolsäure bzw. linolsäurehaltige Gemische können in an sich bekannter Weise gewonnen werden. Einige von Ihnen sind ebenfalls käuflich erhältlich. Beispielhaft seien Sojaöl, Leinsamenöl und vor allem Sonnenblumenöl und Safloröl genannt. Weitere Ausführungen zur Herstellung von Verbindungen der Formel I und insbesondere Verbindungen der Formel IV finden sich beispielsweise in WO 99/29317, US 6,015,833 und US 6,060,514 sowie dem darin genannten Stand der Technik.

Neben den Liponsäure- und CA-Komponenten kann die erfindungsgemäße Behandlung weitere Wirkstoffe miteinbeziehen. Bei diesen wirkstoffen kann es sich insbesondere um solche handeln, deren Wirkung der Liponsäure- bzw. CA-vermittelten Wirkung ähnlich ist oder diese ergänzt. So kann es von Vorteil sein, zusätzlich zur erfindungsgemäßen Kombination, Antioxidantien, Insulin, Antidiabetika, insbesondere die eingangs beschriebenen Hypoglykämika und Antihyperglykämika, und ähnliche Wirkstoffe zu verabreichen. Auch Vitamine, Cofaktoren, Spurenelemente, insbesondere Cr und Zn, Mineralstoffe, Aminosäuren und andere esssentielle Nährstoffe können zweckmäßig sein. Aus praktischen Gründen werden vielfach auch weitere Fettsäuren, gegebenenfalls in Form von Glyceriden, miteinbezogen. Mehrfach ungesättigte Fettsäuren, vor allem ω-3- und $\bar{\omega}$-6-PUFA, z.B. Arachidonsäure und insbesondere Docosahexaensäure und/oder Eicosapentaensäure; Phospholipide, vor allem Phosphatidylcholin, Phosphatidylserin

und Phosphatidylethanolamin; Antioxidantien, vor allem Vitamin E und C, Flavonoide, Tocotrienole, Carnitin etc. werden bevorzugt zusammen mit Liponsäure und Verbindungen der Formel I verabreicht.

Eine besondere Ausführungsform der vorliegenden Erfindung basiert auf der Kombination einer Liponsäure, insbesondere R-Liponsäure, eines physiologisch akzeptablen Derivates oder Salzes davon mit 9c, 11t- und/oder 10t, 12c-Isomeren der Octadecadiensäure, oder physiologisch akzeptablen Derivaten oder Salzen davon.

Die Erfindung beinhaltet im Rahmen therapeutischer Anwendungen, einer Nahrungsergänzung, einer diätetischen Ernährungsstrategie oder im Bereich angereicherter Lebensmittel (functional foods) eine Behandlung von Individuen.

Im Rahmen der Nahrungsergänzung wird die mit der normalen Ernährung gewährleistete Zufuhr an erfindungsgemäßer Wirkstoffkombination ergänzt. In diesem Sinne ist die erfindungsgemäße Wirkstoffkombination auch als Nährstoffkombination zu betrachten. Zweck dieser Nahrungsergänzung kann es sein, entsprechende Ernährungsmängel auszugleichen oder eine über der mit üblicher Ernährung gewährleisteten Menge liegende Zufuhr dieser Wirkstoffe sicherzustellen. So dient die erfindungsgemäße Verwendung zur Nahrungsergänzung auch ernährungsphysiologischen Zwecken, insbesondere der Behandlung entsprechender Mangelerscheinungen bzw. der Veränderung bestimmter Zustände eines Individuums, die mit einer nahrungsergänzenden Zufuhr der erfindungsgemäßen wirkstoffkombination ausgeglichen bzw. bewirkt werden können. Zu den Ausfallerscheinungen und veränderbaren Zuständen gehören die nachfolgend aufgeführten, erfindungsgemäß behandelbaren Störungen bzw. erzielbaren Effekte.

Die erfindungsgemäße Verwendung zu therapeutischen Zwecken betrifft insbesondere die Behandlung von diabetischen Störungen. Darunter versteht man Störungen des Kohlenhydratstoffwechsels. Es handelt sich um ein durch Hyperglykämie gekennzeichnetes Syndrom, das mit einer verminderten Insulinsekretion und/oder Insulinwirkung einhergeht. Hierzu zählen vor allem Diabetes Typ I und der erfindungsgemäß insbesondere behandelbare Diabetes Typ II sowie weitere Störungen diabetogener Genese. Mitunter gehören zu den erfindungsgemäß behandelbaren diabetischen Störungen auch solche Störungen, die zu Diabetes führen können, ohne dass zum Behandlungszeitpunkt ein hyperglykämischer Zustand gegeben sein muß. Hierzu zählen beispielsweise eine Insulinresistenz und eine verminderte Glucosetoleranz. Insbesondere können Zustände einer verminderten Glucose-stimulierten Insulinsekretion behandelt werden.

Zu Störungen diabetogener Ursache gehören vor allem Erkrankungen, die auf hyperglykämische und/oder hyperinsulinämische Zustände zurückzuführen sind. Dies sind vor allem mikro- und makrovaskuläre Komplikationen, die zu Nerven- und Blutgefäßerkrankungen führen, wie Neuropathien, Nephropathien und Retinopathien bzw. Atherosklerose, sowie die darauf zurückzuführenden Folgeerkrankungen, wie Katarakt, Erblindung, Nierenversagen und/oder Amputationen. Zu den erfindungsgemäß behandelbaren Neuropathien gehören insbesondere Polyneuropathien.

Bevorzugte Ausführungsformen der vorliegenden Erfindung richten sich auf die Behandlung von Insulinresistenz, insbesondere Insulinresistenz in Zusammenhang mit Diabetes Typ II.

Eine weitere bevorzugte Ausführungsformen der vorliegenden Erfindung richtet sich auf die Behandlung von Hyperglykämien, insbesondere Insulinresistenz-bedingte.-Hyperglykämien.

Die erfindungsgemäße Verwendung gewinnt bei Erwachsenen mit zunehmendem Lebensalter an Bedeutung. In der Gruppe der über 40-jährigen und vor allem der über 50-jährigen bringt die Behandlung besondere Vorteile mit sich. übergewichtete Individuen stellen eine weitere Gruppe vorteilhaft behandelbarer Individuen dar.

Erfindungsgemäß zu behandelnde Erkrankungen sind häufig gekennzeichnet durch eine progressive Entwicklung, d.h. die vorstehend beschriebenen Zustände verändern sich im Laufe der Zeit, in der Regel nimmt der Schweregrad zu und gegebenenfalls können Zustände ineinander übergehen oder weitere Zustände zu bereits bestehenden Zuständen hinzutreten. Insbesondere nehmen Störungen diabetogener Ursache mit der Dauer hyperglykämischer und/oder hyperinsulinämischer Zustände zu. So stellt die präventive Behandlung von Störungen diabetogener Ursache, insbesondere von Folgeerkrankungen, wie Katarakt, Erblindung, Nierenversagen und Amputation, einen besonders wertvollen Aspekt der erfindungsgemäßen Behandlung dar.

Durch die erfindungsgemäße Verwendung lassen sich eine Vielzahl von Anzeichen, Symptomen und/oder Fehlfunktionen behandeln, die mit den vorstehend genannten Störungen und Zuständen zusammenhängen. Hierzu gehören beispielsweise verschwommenes Sehen, Müdigkeit, Übelkeit, Symptome einer Herzkranzgefäßerkrankung, Hinken, Gangrän, Netzhautablösung oder -hämorrhagie, Katarakt, Erblindung, ein nephrotisches Syndrom, Nierenversagen, sensorische Defizite, Taubheit, schmerzhaftes Stechen oder Brennen, abnorme Gefühlsempfindungen in den Extremitäten, Amputation, Erschöpfungszustände, tiefsitzende Schmerzen, Überempfindlichkeit.

Ein Aspekt einer Verwendung im erfindungsgemäßen Sinne betrifft die Behandlung akuter oder chronischer Störungen, Zustände, Anzeichen, Symptome und/oder Fehlfunktionen, insbesondere von hyperglykämischen und/oder hyperinsulinämischen Zuständen; ein Zweck dieser Behandlung ist eine Behebung der Störungen, Regulation der Zustände, bzw. Linderung der Anzeichen, Symptome und/oder Fehlfunktionen, insbesondere eine Regulation und vor allem Absenkung des Blutglukose- und/oder Blutinsulinspiegels bzw. eine Stimulierung der Glukose-induzierten Insulinsekretion und/oder eine Steigerung der Insulin-vermittelten Glukoseaufnahme. Einem besonderen Aspekt zufolge kann es Zweck der Behandlung sein, die Aktivität des proinflammatorisch wirkenden Redoxfaktors NFKB zu

verringern und/oder Peroxisomen-Proliferator-aktivierte Rezeptoren (PPAR) zu aktivieren. Ein weiterer Aspekt betrifft eine vorbeugende Behandlung (Prophylaxe), insbesondere im Hinblick auf die zuvor genannten Störungen diabetogener Ursache; ein Zweck dieser Behandlung ist es, das Auftreten der Störungen, Zustände, Anzeichen, Symptome und/oder Fehlfunktionen zu vermeiden, wozu auch eine zeitliche Verzögerung des Auftretens zählt. Die Behandlung kann symptomatisch, beispielsweise als Symptomsuppression ausgerichtet sein. Sie kann kurzzeitig erfolgen, mittelfristig ausgerichtet sein, oder es kann sich auch um eine Langzeitbehandlung, beispielsweise im Rahmen einer Erhaltungstherapie, handeln.

Die erfindungsgemäße Verwendung der beschriebenen wirkstoffe beinhaltet im Rahmen der Behandlung ein Verfahren. Dabei wird dem zu behandelnden Individuum, vorzugsweise einem Säuger, insbesondere einem Menschen, und auch einem Nutz- oder Haustier, eine wirksame Menge an Liponsäure-Komponente und eine wirksame Menge an CA-Komponente, in der Regel der pharmazeutischen, tierarzneilichen oder lebensmitteltechnologischen Praxis entsprechend formuliert, verabreicht. Ob eine solche Behandlung angezeigt ist und in welcher Form sie zu erfolgen hat, hängt vom Einzelfall ab und kann sowohl einer fachmännischen medizinischen (in der Regel Fremddiagnose) als auch einer nichtfachmännischen Beurteilung (in der Regel Selbstdiagnose) unterliegen, die vorhandene Anzeichen, Symptome und/oder Fehlfunktionen, Risiken, bestimmte Anzeichen, Symptome und/oder Fehlfunktionen zu entwikkeln, und weitere Faktoren miteinbeziehen kann.

Die Behandlung erfolgt in der Regel durch einmalige oder mehrmalige tägliche Verabreichung gegebenenfalls zusammen oder im Wechsel mit anderen Wirkstoffen oder wirkstoffhaltigen Präparaten, so dass einem zu behandelnden Individuum eine Tagesdosis von etwa 1 mg bis 5 g, vorzugsweise von etwa 10 mg bis 1 g Liponsäure sowie von etwa 10 µg bis 10 g, vorzugsweise von etwa 10 mg bis 5 g wenigstens einer Verbindung der Formel I, bei oraler Gabe, sowie von etwa 5 mg bis 1 g Liponsäure sowie etwa 1 mg bis 1 g wenigstens einer Verbindung der Formel I bei parenteraler Gabe zugeführt wird.

Wirkstoffmengen und -anteile beziehen sich auf den aktiven Wirkstoff, so dass für Salze und Derivate eine entsprechende Umrechnung zu erfolgen hat.

Die Erfindung betrifft auch die Herstellung von Mitteln zur Behandlung eines Individuums, vorzugsweise eines Säugers, insbesondere eines Menschen, Nutz- oder Haustieres.

Ein Gegenstand der vorliegenden Erfindung sind daher auch Mittel, enthaltend

i) wenigstens eine Liponsäure, ein physiologisch akzeptables Derivat oder Salz davon, und
ii) wenigstens eine Verbindung der Formel I, ein physiologisch akzeptables Derivat oder Salz davon, sowie

gegebenenfalls wenigstens einen weiteren Wirkstoff und eine Formulierungsgrundlage.

Erfindungsgemäße Mittel basieren daher auf einer Wirkstoffkombination und gegebenenfalls einer Formulierungsgrundlage.

Zu den Mitteln gehören insbesondere pharmazeutische Mittel, Nahrungsergänzungsmittel und Lebensmittel, insbesondere funktionale oder diätetische Lebensmittel. Die erfindungsgemäßen Lebensmittel besitzen neben einer vorwiegend nährwertbezogenen Funktion zusätzlich eine wirkstoffbezogene Funktion, welche insbesondere die erfindungsgemäße Wirkstoffkombination betrifft. Sie werden daher als funktionale oder diätetische Lebens- oder Nahrungsmittel bezeichnet. Nahrungsergänzungsmittel dienen zur Ergänzung der täglichen Ernährung mit der erfindungsgemäßen wirkstoffkombination, wobei die nährwertbezogene Funktion des Nahrungsergänzungsmittels für sich genommen in den Hintergrund tritt.

Die Wirkstoffkombination im Sinne der Erfindung umfaßt als Wirkstoffkomponente i) wenigstens eine Liponsäure, ein physiologisch akzeptables Derivat oder Salz davon. Gemische dieser Formen sind möglich, jedoch nur in bestimmten Fällen in Betracht zu ziehen. Gemäß einer besonderen Ausführungsform besteht die Wirkstoffkomponente i) aus Liponsäure, vorzugsweise zu mindestens 90 Gew.-% und insbesondere zu mindestens 99 Gew.-% aus dem (R) -Enantiomer, wobei die gewichtsprozentualen Angaben auf das Gesamtgewicht der Wirkstoffkomponente i) bezogen sind.

Weiterhin umfaßt die Wirkstoffkombination im Sinne der Erfindung als Wirkstoffkomponente ii) wenigstens eine Verbindung der Formel I, ein physiologisch akzeptables Derivat oder Salz davon. Bevorzugt sind die oben als besondere Verbindungen der Formel I aufgeführten Wirkstoffe, vor allem Verbindungen der Formel IV. In der Regel handelt es sich um Gemische mehrerer Verbindungen der Formel I. Gemäß einer besonderen Ausführungsform besteht die Wirkstoffkomponente ii) aus 9c,11t- und/oder 10t,12c-Isomeren der Octadecadiensäure.

Weiterhin kann die Wirkstoffkombination im Sinne der Erfindung als Wirkstoffkomponente iii) weitere Wirkstoffe umfassen, beispielsweise die oben in diesem Zusammenhang genannten Wirkstoffe.

Der Anteil der Wirkstoffkombination an der Formulierung ist größer als ein gegebenenfalls in natürlichen Quellen vorhandener Anteil. In diesem Sinne sind die erfindungsgemäßen Mittel, insbesondere Lebensmittel, im Hinblick auf die Wirkstoffkombination angereichert. Der Anteil der Wirkstoffkombination aus i) und ii) an der Formulierung ist vorzugsweise größer als etwa 0,05 Gew.-%, vorteilhafterweise größer als etwa 0,1 Gew.-% und insbesondere größer

als etwa 0,5 Gew.-%. Im Falle eines pharmazeutischen Mittels liegt der Anteil in der Regel bei etwa 1 bis 60 Gew.-%, vorzugsweise bei etwa 5 bis 35 Gew.-% und insbesondere bei etwa 10 bis 30 Gew.-%, im Falle eines Nahrungsergänzungsmittels und vor allem bei Lebensmitteln gegebenenfalls entsprechend niedriger, wenn die Formulierung in größeren Mengen zugeführt wird.

Angaben in Gew.-% beziehen sich, sofern nicht anderes angegeben ist, auf das Gesamtgewicht der Formulierung.

Die Formulierungsgrundlage erfindungsgemäßer Formulierungen enthält physiologisch akzeptable Hilfsstoffe. Physiologisch akzeptabel sind die im Bereich der Pharmazie, der Lebensmitteltechnologie und angrenzenden Gebieten bekanntermaßen verwendbaren Hilfstoffe, insbesondere die in einschlägigen Arzneibüchern (z.B. DAB, Ph. Eur., BP, NF) gelisteten, und auch andere Hilfstoffe, deren Eigenschaften einer physiologischen Anwendung nicht entgegenstehen. Hilfsstoffe im erfindungsgemäßen Sinne können auch einen Nährwert besitzen und deshalb allgemein als Nahrungskomponente verwendet werden. Auch Nährstoffe, insbesondere essentielle Nährstoffe, können dazu gehören.

Geeignete Hilfsstoffe können sein: Netzmittel; emulgierende und suspendierende Mittel; konservierende Mittel; Antioxidantien; Antireizstoffe; Chelatbildner; Dragierhilfsmittel; Emulsionsstabilisatoren; Filmbildner; Gelbildner; Geruchsmaskierungsmittel; Geschmackskorrigentien; Harze; Hydrokolloide; Lösemittel; Lösungsvermittler; Neutralisierungsmittel; Permeationsbeschleuniger; Pigmente; quaternäre Ammoniumverbindungen; Rückfettungs- und Überfettungsmittel; Salben-, Creme- oder Öl-Grundstoffe; Silikon-Derivate; Spreithilfsmittel; Stabilisatoren; Sterilanzien; Suppositoriengrundlagen; Tabletten-Hilfsstoffe, wie Bindemittel, Füllstoffe, Gleitmittel, Sprengmittel oder Überzüge; Treibmittel; Trocknungsmittel; Trübungsmittel; Verdickungsmittel; Wachse; Weichmacher; Weißöle. Eine diesbezügliche Ausgestaltung beruht auf fachmännischem Wissen, wie beispielsweise in Fiedler, H.P., Lexikon der Hilfsstoffe für Pharmazie, Kosmetik und angrenzende Gebiete, 4. Auflage, Aulendorf: ECV-Editio-Cantor-Verlag, 1996, dargestellt ist.

Nahrungskomponenten enthalten in der Regel eine oder mehrere Aminosäuren, Kohlenhydrate oder Fette und sind für die menschliche und/oder tierische Ernährung geeignet. Sie umfassen Einzelkomponenten, häufig pflanzliche aber auch tierische Produkte, insbesondere Zucker gegebenenfalls in Form von Sirups, Fruchtzubereitungen, wie Fruchtsäfte, Nektar, Fruchtpulpen, Pürees oder getrocknete Früchte, beispielsweise Apfelsaft, Grapefruitsaft, Orangensaft, Apfelmus, Tomatensauce, Tomatensaft, Tomatenpüree; Getreideprodukte, wie Weizenmehl, Roggenmehl, Hafermehl, Maismehl, Gerstenmehl, Dinkelmehl, Maissirup, sowie Stärken der genannten Getreide; Milchprodukte, wie Milcheiweiß, Molke, Joghurt, Lecithin und Milchzucker.

Zu den essentiellen Nährstoffen zählen insbesondere Vitamine, Provitamine, Mineralstoffe, Spurenelemente, Aminosäuren und Fettsäuren. Als essentielle Aminosäuren seien genannt Isoleucin, Leucin, Lysin, Methionin, Phenylalanin, Threonin, Tryptophan und Valin. Dazu gehören auch semi-essentielle Aminosäuren, die beispielsweise in Wachstumsphasen oder Mangelzuständen zugeführt werden müssen, wie Glutamin, Arginin, Histidin, Cystein und Tyrosin. Als Spurenelemente seien genannt: essentielle Spurenelemente und Mineralstoffe, deren Notwendigkeit für den Menschen erwiesen ist und deren Mangel zur Manifestation klinischer Symptome führt: Eisen, Kupfer, Zink, Chrom, Selen, Calcium, Magnesium, Kalium, Mangan, Cobalt, Molybdän, Iod, Silicium, Fluor. Ebenso Elemente, deren Funktion für den Menschen noch nicht genügend gesichert ist: Zinn, Nickel, Vanadium, Arsen, Lithium, Blei, Bor. Als für den Menschen essentielle Fettsäuren seien genannt: Linolsäure und Linolensäure, ARA (Arachidonsäure) und DHA (Docosahexaensäure) für Säuglinge und möglicherweise EPA (Eicosapentaensäure) und DHA auch für Erwachsene. Eine umfassende Aufzählung von Vitaminen findet sich in "Referenzwerte für die Nährstoffzufuhr", 1. Auflage, Umschau Braus Verlag, Frankfurt am Main, 2000, herausgegeben von der Deutschen Gesellschaft für Ernährung.

Die Summe aus Wirkstoffkomponente und Formulierungsgrundlage beträgt in der Regel 100 Gew.-%.

Beispiele geeigneter Formulierungen zur Nahrungsergänzung sind Kapseln, Tabletten, Pillen, Pulverbeutel, Flüssigampullen und Fläschchen Mit Tropfeinsätzen, im übrigen die nachfolgend genannten Arzneiformen.

Beispiele geeigneter pharmazeutischer Formulierungen sind feste Arzneiformen, wie Pulver, Puder, Granulate, Tabletten, insbesondere Filmtabletten, Pastillen, Sachets, Cachets, Dragees, Kapseln wie Hart- und Weichgelatinekapseln, Suppositorien oder vaginale Arzneiformen, halbfeste Arzneiformen, wie Salben, Cremes, Hydrogele, Pasten oder Pflaster, sowie flüssige Arzneiformen, wie Lösungen, Emulsionen, insbesondere Öl-in-wasser-Emulsionen, Suspensionen, beispielsweise Lotionen, Injektions- und Infusionszubereitungen, Augen- und Ohrentropfen. Auch implantierte Abgabevorrichtungen können zur Verabreichung erfindungsgemäßer Wirkstoffe verwendet werden. Ferner können auch Liposomen oder Mikrosphären zur Anwendung kommen.

Lebensmitteltechnische Formulierungen haben in der Regel die übliche Form und werden bevorzugt in Form von Kleinkindnahrung, Frühstückszubereitungen, vor allem in Form von Müslis oder Riegeln, Sportlerdrinks, Komplettmahlzeiten, insbesondere im Rahmen von total bilanzierten Diäten, diätetische Zubereitungen, wie Diätdrinks, Diätmahlzeiten und Diätriegel, angeboten.

Die Formulierungen werden vorzugsweise auf oralem, sie können aber auch insbesondere im Bereich der Arzneimittel auf rektalem, transdermalem, subkutanem, intravenösem, intramuskulärem oder intranasalem Weg verabreicht werden.

Bei der Herstellung der Zusammensetzungen werden die Wirkstoffe gewöhnlich mit einem geeigneten Hilfsstoff, in diesem Fall auch als Exzipient zu bezeichnen, vermischt oder verdünnt. Exzipienten können feste, halbfeste oder flüssige Materialien sein, die als Vehikel, Träger oder Medium für den Wirkstoff dienen. Die Zumischung weiterer Hilfsstoffe erfolgt erforderlichenfalls in an sich bekannter Weise. Es können Formgebungsschritte, gegebenenfalls in Verbindung mit Mischvörgangen, durchgeführt werden, z.B. eine Granulierung, Komprimierung und ähnliches.

Insbesondere können die Wirkstoffkomponenten gemeinsam formuliert werden. Sie können aber auch zunächst getrennt verarbeitet und anschließend in einer kompartimentierten, z.B. mehrschichtigen Arzneiform zusammengeführt werden. Dadurch kann möglichen Wirkstoffinkompatibilitäten und unterschiedlichen wirkstoffeigenschaften, wie Bioverfügbarkeit, Stabilität, Löslichkeit und ähnlichem, Rechnung getragen werden.

Ein weiterer Gegenstand der vorliegenden Erfindung sind Mittel in Form einer Handelspackung mit wenigstens einem Mittel auf Basis

i) wenigstens einer Liponsäure, eines physiologisch akzeptablen Derivates oder Salzes davon, und/oder
ii) wenigstens einer Verbindung der Formel I, eines physiologisch akzeptablen Derivates oder Salzes davon,

gegebenfalls mit Instruktionen für die therapeutische Verwendung von Liponsäuren, physiologisch akzeptablen Derivaten oder Salzen davon in Kombination mit Verbidnungen der Formel I, physiologisch akzeptablen Derivaten oder Salzen davon.

Eine Ausführungsform dieses erfindungsgemäßen Gegenstandes betrifft Handelspackungen mit wenigstens einem, insbesondere pharmazeutischen, Mittel der oben beschriebenen Art mit einer erfindungsgemäßen Wirkstoffkombination. Unter diese Ausführungsform fallen auch Handelspackungen mit mehreren Kombinationspräparaten in unterschiedlichen Dosierungen oder Formulierungen. Handelspakkungen dieser Ausführungsform beinhalten demnach die Wirkstoffkomponenten i) und ii) in gemeinsamer Formulierung.

Eine weitere Ausführungsform betrifft Handelspackungen mit zwei oder mehreren, räumlich voneinander getrennten, insbesondere pharmazeutischen, Mitteln, von denen wenigstens zwei Mittel verschiedene Wirkstoffe umfassen. Bei diesen Mitteln kann es sich insbesondere um Monopräparate handeln, also vor allem solche mit Wirkstoffkomponente i) oder ii). In diesen Fällen beinhaltet die Handelspackung Instruktionen im Sinne der Erfindung für die kombinierte Verwendung der i) bzw. ii) umfassenden Mittel. Handelspackungen dieser Ausführungsform beinhalten demnach die Wirkstoffkomponenten i) und/oder ii) in getrennter Formulierung, d.h. in Form von wenigstens zwei räumlich voneinander getrennten Mitteln.

Eine weitere Ausführungsform betrifft Handelspackungen mit wenigstens einem, insbesondere pharmazeutischen, Mittel auf Basis

i) wenigstens einer Liponsäure, eines physiologisch akzeptablen Derivates oder Salzes davon; oder
ii) wenigstens einer Verbindung der Formel I, eines physiologisch akzeptablen Derivates oder Salzes davon.

Hierbei handelt es sich um Monopräparate. In diesen Fällen beinhaltet die Handelspackung Instruktionen im Sinne der Erfindung für die therapeutische Verwendung des Mittels in Kombination mit den übrigen die erfindungsgemäße Wirkstoffkombination bildenden Wirkstoffen, die nicht Teil der Handelspackung sind, in Form wenigstens eines weiteren Mittels. Handelspackungen dieser Ausführungsform beinhalten demnach einen Teil der erfindungsgemäßen Wirkstoffkombination. Der nicht enthaltene Teil wird als Teil beiliegender Instuktionen bestimmungsgemäß einbezogen.

Es versteht sich, dass erfindungsgemäße Handelspackungen auch weitere Präparate, insbesondere wirkstoffhaltige Formulierungen, sowie umfassende auch über den vorstehend genannten Inhalt hinausgehende Instruktionen enthalten können.

Die vorliegende Erfindung wird anhand der nachfolgenden Beispiele näher erläutert, ohne darauf beschränkt zu sein.

Beispiel 1

Pharmazeutische Mittel

a) Weichgelatine-Kapsel mit Liponsäure und CLA

| (Liponsäure 200 mg + CLA 500 mg) | |
|---|---|
| CLA | 500 mg |
| Liponsäure | 200 mg |
| D/L-alpha-Tocopherol | 60 mg |

Beispiel 2

Funktionelles Nahrungsmittel

a) Riegel mit Liponsäure und CLA

| (CLA 1000 mg + 400 mg Liponsäure / Riegel (60 g)) | |
|---|---|
| CLA | 1000 mg |
| Liponsäure | 400 mg |
| D/L-alpha-Tocopherol | 150 mg |
| Sirup aus Fructose | 4,2 g |
| Glucose | 12 g |
| Gebräuntem Zucker | 3 g |
| Glycerin | 3 g |
| Lecithin Lecithin | 125 mg |
| Pflanzenöl Gehärtetes Pflanzenöl | 1,2 g |
| Haferflocken | 17,975 g |
| Puffreis Puffreis | 7 g |
| Geröstete und gehackte Mandeln | 5,6 g |
| Kokosflocken | 4 g |

b) Müsli mit Liponsäure und CLA

| (CLA 500 mg + Liponsäure 250 mg / 100 g Müsli) | |
|---|---|
| Haferflocken | 40 g |
| Weizenflocken | 27 g |
| Rosinen | 13 g |
| Getrocknete Apfelscheiben | 6 g |
| Getrocknete Aprikosen | 3 g |
| Weizenkeime | 3 g |
| Geröstete und gemahlene Haselnüsse | 6 g |
| Angereichertes Milchpulver, enthaltend | 7 g |
| CLA | 500 mg |
| Liponsäure | 250 mg |
| D/L-alpha-Tocopherol | 60 mg |
| Lecithin | 200 mg |

Beispiel 3

Biologische Wirkung

Die fettleibige Zucker-Ratte (fa/fa) stellt ein Tiermodell für Insulinresistenz, Glukose-Intoleranz, Hyperinsulinämie und Dyslipidämie dar. Mithilfe dieses Modells wurde der Effekt von 4 verschiedenen Ernährungsformen (Kontrolle, R-Liponsäure (R-LA), konjugierte Linolsäure (CLA) und eine Kombination aus konjugierter Linolsäure und R-Liponsäure) auf die Insulin-induzierte Glukosetransportaktivität im Skelettmuskel und auf die Glukosetoleranz bestimmt.

Die Versuchstiere wurden bei unbegrenzt verfügbarem Futter und Wasser gehalten. Im Alter von 9 bis 10 Wochen wurden die Tiere entweder der mit Placebo gefütterten Kontrollgruppe oder einer der Testgruppen zugeordnet, die entweder R-Liponsäure (10 mg/kg Körpergewicht), konjugierte Linolsäure (c9,t11:t10,c12; 50:50; 0,3 g/kg Körpergewicht) oder beides erhielten. R-Liponsäure wurde durch intraperitoneale Injektion und kunjugierte Linolsäure über eine Schlundsonde verabreicht. Die Behandlungszeit betrug 18 bis 20 Tagen.

24 Stunden nach der ersten Behandlung wurden die Versuchstiere mit Natriumpentobarbital (50 mg/kg ip) anästhesiert, nachdem man während der vorhergehenden 13 Stunden das Futter auf 4 g reduziert hatte. Der rechte Soleus und sowohl rechter als auch linker Epitrochlearis wurden chirurgisch entfernt und für die Inkubation in vitro präpariert. Die Präparation der Muskeln ist in Peth JA et al. (Am J Physiol Regulatory Integrative Comp Physiol 2000; 278: R453-R459) beschrieben. Die Glukosetransportaktivität wurde dann in der von Hendriksen und Halseth (J Appl Physiol 1994; 76: 1862-1867) beschriebenen Art und Weise bestimmt.

18 Tage nach Behandlung wurde an sämtlichen Tieren ein oraler Glukosetoleranztest durchgeführt. 12 Stunden vor dem Test wurden die Ratten auf 4 g Futter gesetzt. Allen Ratten wurde 1g/kg Glukose über eine Schlundsonde verabreicht. Aus einem Schnitt an der Schwanzspitze wurden Blutproben entnommen, und zwar zu den Zeitpunkten 0, 30, 60, 90 und 120 min nach Glukoseaufnahme. Die Plasmaproben wurden auf Glukose und freie Fettsäuren spektrophotometrisch und auf Insulin durch Radioimmunoassay untersucht.

Die Aufnahme von 2-Deoxyglukose in den Epitrochlearis bzw. Soleus ist in Tabelle 1 gezeigt. Ohne Insulin-Stimulierung unterschied sich die Aufnahme von 2-Deoxyglukose durch die Muskeln der Kontrollgruppe nicht von den Muskeln der Testgruppen. Wurde die Glukosetransportaktivität der Skelettmuskel allerdings durch Insulin in vitro stimuliert, konnten deutliche Unterschiede bezüglich der durch Insulin stimulierten Zunahme der Glukosetransportaktivität beobachtet werden. So nahm die 2-Deoxyglukose-Aufnahme in den Epitrochlearis und den Soleus ausgehend von einem Grundwert von 120 bzw. 220 auf 278 bzw. 579 pmol/mg Muskel 20 min nach Stimulation mit Insulin zu, wenn die Tiere mit einer Kombination aus R-Liponsäure und konjugierter Linolsäure gefüttert worden waren. Diese Wirkung wurde nur in Tieren beobachtet, die die erfindungsgemäße Kombination erhalten hatten. Weder R-Liponsäure noch konjugierte Linolsäure allein waren in der Lage, die Glukosetransportaktivität des Skelettmuskels nach Stimulation mit Insulin über das in der Kontrollgruppe beobachtete Maß hinaus zu steigern.

Tabelle 1:

| 2-Deoxyglukose-Spiegel in pmol/mg Muskel | | | |
|---|---|---|---|
| Behandlungsgruppe | Basal | + Insulin [5 mU/ml] | Zunahme |
| Epitrochlearis | | | |
| Kontrolle | 127 +/- 3 | 220 +/- 8 | 93 +/- 8 |
| 10 mg/kg R-LA | 123 +/- 3 | 225 +/- 8 | 102 +/- 11 |
| 0,3 g/kg CLA | 126 +/- 4 | 248 +/- 14 | 123 +/- 13 |
| 10 mg/kg R-LA + 0,3 g/kg CLA | 120 +/- 7 | 278 +/- 12 | 158 +/- 6 |
| Soleus | | | |
| Kontrolle | 229 +/- 5 | 469 +/- 13 | 240 +/- 11 |
| 10 mg/kg R-LA | 224 +/- 7 | 481 +/- 19 | 258 +/- 16 |
| 0,3 g/kg CLA | 231 +/- 6 | 492 +/- 9 | 261 +/- 13 |
| 10 mg/kg R-LA + 0,3 g/kg CLA | 220 +/- 9 | 579 +/- 40 | 359 +/- 35 |

## Patentansprüche

**1.** Verwendung wenigstens einer Liponsäure, eines physiologisch akzeptablen Derivates oder Salzes davon, und wenigstens einer Verbindung der Formel I

$$H_3C\text{-}(CH_2)_{n1}\text{-}(CH{=}CH)_{n3}\text{-}(CH_2)_{n2}\text{-}COOH \qquad I$$

worin n1, n2 unabhängig voneinander den Wert einer ganzen Zahl von 3 bis 9 haben, n3 den Wert einer ganzen Zahl von 2 bis 6 hat und die Zahl der Kohlenstoffatome 18, 20 oder 22 beträgt, eines physiologisch akzeptablen Derivates oder Salzes davon, zur Herstellung eines Mittels zur Behandlung diabetischer Störungen.

**2.** Verwendung nach Anspruch 1, wobei die Verbndung der Formel I eine Verbindung der Formel IV

$$H_{3C}\text{-}(CH_2)_{n1}\text{-}CH{=}CH\text{-}CH{=}CH\text{-}(CH_2)_{n2}\text{-}COOH \qquad IV$$

ist, worin n1, n2 unabhängig voneinander den Wert einer ganzen Zahl von 3 bis 9 haben und die Summe aus (n1+n2)=12 beträgt.

**3.** Verwendung nach Anspruch 1 oder 2, wobei n1 = 3, 4 oder 5 ist und n2 = 7, 8 oder 9 ist.

**4.** Verwendung nach Anspruch 1, wobei die Verbindung der Formel I ausgewählt ist unter 9c,11t-Octadecadiensäure und 10t,12c-0ctadecadiensäure.

**5.** Verwendung nach einem der vorhergehenden Ansprüche zur Behandlung von Diabetes Typ II.

**6.** Verwendung nach einem der vorhergehenden Ansprüche zur Behandlung von Insulinresistenz.

**7.** Verwendung nach einem der vorhergehenden Ansprüche zur Behandlung von Hyperglykämie.

**8.** Verwendung nach einem der vorhergehenden Ansprüche zur Behandlung mikro- und makrovaskulärer Störungen diabetogener Genese.

**9.** Verwendung nach Anspruch 8 zur Behandlung diabetischer Neuropathy, diabetischer Nephropathy, diabetischer Retinopathy, Atherosklerose.

**10.** Verwendung nach einem der vorhergehenden Ansprüche zur präventiven Behandlung von Katarakt, Erblindung, Nierenversagen und Amputationen diabetogener Genese.

**11.** Mittel, enthaltend

i) wenigstens eine Liponsäure, ein physiologisch akzeptables Derivat oder Salz davon, und
ii) wenigstens eine Verbindung der Formel I

$$H_3C\text{-}(CH_2)_{n1}\text{-}(CH{=}CH)_{n3}\text{-}(CH_2)_{n2}\text{-}COOH \qquad I$$

worin n1, n2 unabhängig voneinander den Wert einer ganzen Zahl von 3 bis 9 haben, n3 den Wert einer ganzen Zahl von 2 bis 6 hat und die Zahl der Kohlenstoffatome 18, 20 oder 22 beträgt, ein physiologisch akzeptablen Derivat oder Salz davon, sowie

gegebenenfalls wenigstens einen weiteren Wirkstoff und eine Formulierungsgrundlage.

**12.** Mittel nach Anspruch 11, **dadurch gekennzeichnet, daß** der wirkstoffanteil größer als 0,05 Gew.-% ist.

**13.** Handelspackung mit wenigstens einem Mittel auf Basis

i) wenigstens einer Liponsäure, eines physiologisch akzeptablen Derivates oder Salzes davon, und
ii) wenigstens einer Verbindung der Formel I

$$H_3C\text{-}(CH_2)_{n1}\text{-}(CH{=}CH)_{n3}\text{-}(CH_2)_{n2}\text{-}COOH \qquad I$$

worin n1, n2 unabhängig voneinander den Wert einer ganzen Zahl von 3 bis 9 haben, n3 den Wert einer ganzen Zahl von 2 bis 6 hat und die Zahl der Kohlenstoffatome 18, 20 oder 22 beträgt, eines physiologisch akzeptablen Derivates oder Salzes davon, sowie

mit Instruktionen für die therapeutische Verwendung von Liponsäuren, physiologisch akzeptablen Derivaten oder Salzen davon in Kombination mit Verbindungen der Formel I, physiologisch akzeptablen Derivaten oder Salzen davon.

## Claims

**1.** The use of at least one lipoic acid, a physiologically acceptable derivative or salt thereof and at least one compound of the formula I

$$H_3C\text{-}(CH_2)_{n1}\text{-}(CH{=}CH)_{n3}\text{-}(CH_2)_{n2}\text{-}COOH \qquad I$$

in which n1, n2 have, independently of one another, the value of an integer from 3 to 9, n3 has the value of an integer from 2 to 6, and the number of carbon atoms is 18, 20 or 22, a physiologically acceptable derivative or salt thereof, for the preparation of an agent for the treatment of diabetic disorders.

**2.** The use as claimed in claim 1, wherein the compound of formula I is a compound of the formula IV

$$H_3C\text{-}(CH_2)_{n1}\text{-}CH{=}CH\text{-}CH{=}CH\text{-}(CH_2)_{n2}\text{-}COOH \qquad IV$$

in which n1, n2 have, independently of one another, the value of an integer from 3 to 9, and the total of (n1+n2) is 12.

**3.** The use as claimed in claim 1 or 2 wherein n1 is 3, 4 or 5 and n2 is 7, 8 or 9.

**4.** The use as claimed in claim 1, where the compound of the formula IV is selected from 9c,11t-octadecadienoic acid and 10t,12c-octadecadienoic acid.

**5.** The use as claimed in any of the preceding claims for the treatment of type II diabetes.

**6.** The use as claimed in any of the preceding claims for the treatment of insulin resistance.

**7.** The use as claimed in any of the preceding claims for the treatment of hyperglycemia.

**8.** The use as claimed in any of the preceding claims for the treatment of microvascular and macrovascular disorders of diabetic origin.

**9.** The use as claimed in claim 8 for the treatment of diabetic neuropathy, diabetic nephropathy, diabetic retinopathy, atherosclerosis.

**10.** The use as claimed in any of the preceding claims for the preventive treatment of cataract, blindness, renal failure and amputations of diabetic origin.

**11.** A composition comprising

i) at least one lipoic acid, a physiologically acceptable derivative or salt thereof, and

ii) at least one compound of the formula I

$$H_3C-(CH_2)_{n1}-(CH=CH)_{n3}-(CH_2)_{n2}-COOH \qquad I$$

in which n1, n2 have, independently of one another, the value of an integer from 3 to 9, n3 has the value of an integer from 2 to 6, and the number of carbon atoms is 18, 20 or 22, a physiologically acceptable derivative or salt thereof, and

where appropriate at least one active ingredient and a formulation base.

**12.** A composition as claimed in claim 11, wherein the active ingredient content is greater than 0.05% by weight.

**13.** Commercial pack having at least one composition based on

i) at least one lipoic acid, a physiologically acceptable derivative or salt thereof, and
ii) at least one compound of the formula I

$$H_3C-(CH_2)_{n1}-(CH=CH)_{n3}-(CH_2)_{n2}-COOH \qquad I$$

in which n1, n2 have, independently of one another, the value of an integer from 3 to 9, n3 has the value of an integer from 2 to 6, and the number of carbon atoms is 18, 20 or 22, a physiologically acceptable derivative or salt thereof, and

with instructions for the therapeutic use of lipoic acids, physiologically acceptable derivatives or salts thereof in combination with compounds of the formula I, physiologically acceptable derivatives or salts thereof.

## Revendications

**1.** Utilisation d'au moins un acide liponique, d'un dérivé ou sel d'un tel acide acceptable pour l'usage pharmaceutique, et d'au moins un composé de formule I :

$$H_3C-(CH_2)_{n1}(CH=CH)_{n3}-(CH_2)_{n2}-COOH \qquad I$$

dans laquelle n1 et n2 sont des nombres entiers indépendants l'un de l'autre allant de 3 à 9, n3 est un nombre entier allant de 2 à 6 et le nombre des atomes de carbone est de 18, 20 ou 22, d'un dérivé ou sel d'un tel composé acceptable pour l'usage pharmaceutique, pour la préparation d'un produit pour le traitement des troubles diabétiques.

**2.** Utilisation selon la revendication 1, dans laquelle le composé de formule I est en fait un composé de formule IV :

$$H_3C-(CH_2)_{n1}-CH=CH-CH=CH-(CH_2)_{n2}-COOH \qquad IV$$

dans laquelle n1 et n2, indépendants l'un de l'autre, sont des nombres entiers allant de 3 à 9 et la somme (n1+n2) est égale à 12.

**3.** Utilisation selon la revendication 1 ou 2, dans laquelle n1 = 3, 4 ou 5 et n2 = 7, 8 ou 9.

**4.** Utilisation selon la revendication 1, dans laquelle le composé de formule I est choisi parmi l'acide 9c, 11t-octadécadiénoïque et l'acide 10t, 12c-octadécadiénoïque.

**5.** Utilisation selon l'une des revendications qui précèdent pour le traitement du diabète de type II.

6. Utilisation selon l'une des revendications qui précèdent pour le traitement de la résistance à l'insuline.

7. Utilisation selon l'une des revendications qui précèdent pour le traitement de l'hyperglycémie.

8. Utilisation selon l'une des revendications qui précèdent pour le traitement des troubles micro- et macro-vasculaires à genèse diabétogène.

9. Utilisation selon la revendication 8, pour le traitement de la neuropathie diabétique, de la néphropathie diabétique, de la rétinopathie diabétique, de l'athérosclérose.

10. Utilisation selon l'une des revendications qui précèdent pour le traitement préventif de la cataracte, de la cécité, des troubles rénaux et des amputations à genèse diabétogène.

11. Produit contenant

i) au moins un acide liponique, un dérivé ou sel d'un tel acide acceptable pour l'usage pharmaceutique, et
ii) au moins un composé de formule I

$$H_3C\text{-}(CH_2)_{n1}\text{-}(CH{=}CH)_{n3}\text{-}(CH_2)_{n2}\text{-}COOH \qquad I$$

dans laquelle n1 et n2 sont, indépendamment l'un de l'autre, des nombres entiers allant de 3 à 9, n3 est un nombre entier allant de 2 à 6 et le nombre des atomes de carbone est de 18, 20 ou 22, un dérivé ou sel d'un tel composé acceptable pour l'usage pharmaceutique, et

le cas échéant, au moins une autre substance active et une base de formulation.

12. Produit selon la revendication 11, **caractérisé en ce que** la teneur en substance active est supérieure à 0,05 % en poids.

13. Emballage commercial contenant au moins un produit à base de :

i) au moins un acide liponique, un dérivé ou sel d'un tel acide acceptable pour l'usage pharmaceutique, et
ii) au moins un composé de formule I :

$$H_3C\text{-}(CH_2)_{n1}\text{-}(CH{=}CH)_{n3}\text{-}(CH_2)_{n2}\text{-}COOH \qquad I$$

dans laquelle n1 et n2 sont, indépendamment l'un de l'autre, des nombres entiers allant de 3 à 9, n3 est un nombre entier allant de 2 à 6 et le nombre des atomes de carbone est de 18, 20 ou 22, un dérivé ou sel d'un tel composé acceptable pour l'usage pharmaceutique, et

des instructions pour l'utilisation thérapeutique des acides liponiques, des dérivés ou sels de ces acides acceptables pour l'usage pharmaceutique en combinaison avec des composés de formule I, leurs dérivés ou sels acceptables pour l'usage pharmaceutique.